(19) Europäisches Patentamt
European Patent Office
Office européen des brevets

(11) **EP 0 555 549 B1**

(12) **EUROPÄISCHE PATENTSCHRIFT**

(45) Veröffentlichungstag und Bekanntmachung des Hinweises auf die Patenterteilung:
**28.02.1996 Patentblatt 1996/09**

(51) Int. Cl.$^6$: **A61B 17/32**, A61B 17/39, A61M 1/00, F04B 43/08

(21) Anmeldenummer: **92121670.1**

(22) Anmeldetag: **19.12.1992**

(54) **Verfahren und Vorrichtung zum Hochdruckflüssigkeitsschneiden**

Method and apparatus for high pressure liquid-jet-cutting

Procédé et appareil de découpage par jet de liquide à haute pression

(84) Benannte Vertragsstaaten:
**CH DE ES FR GB IT LI NL**

(30) Priorität: **16.01.1992 DE 9200452 U**

(43) Veröffentlichungstag der Anmeldung:
**18.08.1993 Patentblatt 1993/33**

(73) Patentinhaber: **DORNIER MEDIZINTECHNIK GMBH**
**D-82101 Germering (DE)**

(72) Erfinder: **Rau, Horst-Günter, Dr. med.**
**W-8000 München 71 (DE)**

(74) Vertreter: **Landsmann, Ralf, Dipl.-Ing.**
**D-88039 Friedrichshafen (DE)**

(56) Entgegenhaltungen:
EP-A- 0 258 901          EP-A- 0 280 972
EP-A- 0 411 170          DE-A- 3 715 418
DE-A- 4 018 736          US-A- 2 112 145

• PATENT ABSTRACTS OF JAPAN vol. 14, no. 146 (C-704)20. März 1990 & JP-A-20 17 053 (TATSUGUCHI MIKIO)

## Beschreibung

Die Erfindung betrifft ein Verfahren sowie eine Vorrichtung zum Hochdruckflüssigkeitsschneiden organischer Gewebe, insbesondere für die Trennung parenchymatöser Gewebe.

Stand der Technik:

Die heutigen Leberresektionsverfahren beruhen auf einer Unterteilung der Leber in verschiedene Lappen und Segmente. Diese hält sich an die Verzweigungen der zuführenden Gefäße im Bereich des Portalfeldes sowie an die Lokalisation der abführenden Lebervenen. Bei der Resektion von Lebermetastasen und bei primären Lebertumoren gilt es, den Tumor so parenchymsparend wie möglich im Gesunden zu entfernen. Hierbei sind folgende wesentliche Probleme zu bewältigen:

1. Die Resektion soll unter möglichst geringem Blutverlust erfolgen.
2. Die Resektionszeit soll möglichst kurz sein.
3. Die zuführenden Blutgefäße (Leberarterie und Pfortader) sollen möglichst kurz oder gar nicht abgeklemmt werden, um die für das Organ schädliche Warmischämiezeit möglichst kurz zu halten oder zu vermeiden.
4. Bei der Resektion zentraler Segmente dürfen die versorgenden und abführenden Gefäße und Gallengänge nachgeschalteter Segmente nicht verletzt werden.

Die bislang geübte Fingerfraktion-Technik kann diesen erhöhten Ansprüchen nicht mehr gerecht werden. Hierbei wird das Parenchym zwischen den Fingern zerquetscht, um die größeren Gefäße zu isolieren und zu ligieren (Pachter 1983). Aus diesem Grund finden in der Leberchirurgie immer mehr technische Hilfsmittel Anwendung. Neben den thermischen Verfahren, wie dem Laser und dem Hochfrequenzelektrokauter, die wegen der fehlenden Möglichkeit zwischen Parenchym und Gefäßstrukturen zu diskriminieren und deshalb heute kaum eine Rolle spielen, hat der Ultraschallaspirator an Bedeutung gewonnen (Hodgson 1979, 1984, Scheele 1989). Hierbei wird eine, mit einer Absaugung versehene Metallröhre, mit Hilfe des Ultraschalls in axiale Schwingung versetzt. Wegen des unterschiedlichen Gewebewiderstandes ist mit dieser Technik ein selektives Schneiden, das heißt eine Trennung der Gefäße vom Parenchym, möglich. Die widerstandsfähigeren Gefäße können dann bei guter Übersicht einzeln gefasst und ligiert werden.Dadurch lässt sich erwiesenermaßen der Blutverlust reduzieren. Die Schnittgeschwindigkeit ist allerdings gering, so daß die Operationszeiten gegenüber der Fingerfracture Technik verlängert sind.

Bei der Jet-cutting Technik wird über eine Hochdruckpumpe einer Flüssigkeit eine Potentielle Energie verliehen. Über eine Hochdruckleitung gelangt die Flüssigkeit an eine Düse, an der die potentielle Energie in kinetische umgewandelt wird. Zwischen dem Flüssigkeitsdruck p und der Austrittsgeschwindigkeit des Flüssigkeitsstrahls v besteht die Beziehung

$$v = \mu^* \sqrt{[2p/q]}.$$

Dabei ist q die Viskosität der Flüssigkeit. Die Verluste, die bei der Energieumwandlung entstehen, sind wesentlich durch die von der Düsengeometrie bestimmten Ausflusszahl $\mu$ festgelegt. Somit ist diese ein Maß für das Verhältnis zwischen tatsächlicher und theoretisch erreichbarer Strahlgeschwindigkeit.

Die Ausbildung eines divergenten Flüssigkeitsstrahls an der Luft wurde von YANAIDA 1980 beschrieben: In einer Kernzone besteht ein kompakter, turbulenter Flüssigkeitsstrahl der sich mit zunehmender Entfernung in einzelne Flüssigkeitsballen und dann Tropfen zerlegt. Im kontinuierlichen Strahlbereich bleibt die Axialgeschwindigkeit nahezu konstant, im tropfenförmigen Bereich verringert sie sich, bis sie im Auflösungsbereich abrupt absinkt.

Für das Schneiden mit dem Wasserstrahl (Jet-Cutting) im parenchymatösen Gewebe haben sich Druckwerte von 10-50 bar bei Düsen von 0.05-0.2 mm Durchmesser bewährt (Bengmark 1987, Papachristou 1982, Une 1989, Rau 1990). Hierbei wird das weichere Leberparenchym von den härteren Gefäßen und Gallengängen abgespült. Dadurch kommen diese Strukturen deutlich zur Darstellung und können wie bei dem Ultraschallaspirator isoliert gefasst und ligiert werden (Bengmark 1987, Papachristou 1582, Une 1989). Der Vorteil dieser Technik liegt in der deutlich höheren Schnittgeschwindigkeit bei gleicher Schnittqualität (Rau 1989, 1990). Zudem ermöglicht diese Technik ein berührungsfreies Arbeiten.Nicht zuletzt ist diese Technik einfach und billiger als die Ultraschallaspiratortechnik.

Auf dem Gebiet der Koagulation ist ein Argon beam der FA.Erbe bekannt, bei dem ein Argongas über eine Hochfrequenz ionisiert wird und diese Energie so zur Koagulation auf das Gewebe übertragen werden kann. Mit dieser Technik ist allerdings ein Schneiden nicht möglich.

Die Applikation der Hochfrequenz zur kontaktfreien Koagulation wurde beschrieben (Reidenbach, 1988). Hierbei wurde eine NaCl Lösung als Leitungsüberträger verwendet. Dieses Verfahren dient jedoch lediglich dazu, daß ein Verkleben der üblichen Metallelektroden mit dem zu koagulierenden Gewebe verhindert wird. Die selektive Schneidewirkung eines Hochdruckstrahls findet hierbei keinerlei Berücksichtigung. Gerade hieraus ergeben sich aber wesentliche Unterschiede.

Aus der EP 280 972 A1 ist eine Flüssigkeitsstrahl-Schneidevorrichtung bekannt, deren Handstück neben der Schneide-Düse, an dem der Schneidestrahl austritt, eine Koagulationselektrode umfaßt. Diese Vorrichtung dient lediglich dazu, das Hantieren beim Wechsel von Schneiden/Koagulieren während der Operation zu vereinfachen. Schneiden und Koagulieren erfolgen zeitlich versetzt und unabhängig voneinander nach jeweils bekannten Methoden.

Die beschriebenen Jet-cutting Geräte (Bengmark, Une, Papachristou, Beer) verwenden Kolbenpumpsysteme, die eine vollständige Trennung der Schneidelösung von dem Pumpensystem nicht zulassen.

Mittlerweile hat die Jet-cutting Technik klinischen Einsatz gefunden und es konnten die in Experimenten gewonnenen Erfahrungen bestätigt werden (Bengmark 1987, Papachristou 1982, Une 1989, Rau 1990). Dennoch sind die Möglichkeiten dieser Technik nicht ausgeschöpft. Aufgabe der vorgelegten Erfindung ist es, die bekannten Geräte und Verfahren dahingehend zu verbessern, daß eine höhere Schnittgeschwindigkeit sowie eine Verbesserung der Schnittqualität erzielt wird. Diese Aufgabe wird durch ein Verfahren nach Anspruch 1 sowie eine Vorrichtung nach Anspruch 7 gelöst. Vorteilhafte Ausbildungen sind Gegenstand weiterer Ansprüche.

Erfindungsgemäß wird das bekannte Jet-Cutting mit der Hochfrequenzkoagulation kombiniert, und zwar derart, daß dem Flüssigkeitsschneidestrahl eine elektrische Hochfrequenz überlagert wird. Dadurch ist bei selektiver Schneidewirkung eine zusätzliche Koagulation kleiner Gefäße möglich, wodurch ein nahezu blutungsfreier Schnitt erreicht wird. Der wesentliche Vorteil der Kombination dieser beiden Techniken beinhattet die Erhöhung der Schnittgeschwindigkeit und eine deutliche Verminderung des Blutverlustes und damit eine Minimierung der Belastung des Patienten.

Voraussetzung für die Ausführung der erfindungsgemäßen Kombination von Jet-Cutting und Hochfrequenzkoagulator ist die vollständige elektrische und hygienische Trennung der Schneideflüssigkeit von der Druckerzeugungsvorrichtung. Deshalb war die Entwicklung spezieller Druckerzeugungsvorrichtungen erforderlich.

Damit dem Schneidestrahl eine elektrische Hochfrequenz aufgeprägt werden kann, muß als Schneideflüssigkeit eine elektrisch leitfähige Flüssigkeit verwendet werden. Bevorzugt wird eine physiologische Elektrytlösung oder eine hypertone Lösung verwendet. Vorteilhaft wird der Schneideflüssigkeit ein Zusatz beigefügt, der die Oberflächenspannung senkt, um so die Austrittsgeschwindigkeit zu erhöhen.

In einer vorteilhaften Ausführung wird die Schneideflüssigkeit vor Düsenaustritt erwärmt. Dadurch kann ebenfalls die Oberflächenspannung erhöht werden. Darüberhinaus kann durch die Erwärmung bei geeigneter Auswahl der Parameter für die Ausbildung des Flüssigkeitsstrahls bereits eine thermische Koagulation erzielt werden oder der Kolagulationseffekt der dem Schneidestrahl überlagerten Hochfrequenz noch verstärkt werden.

Bei Verwendung einer 5-20% NaCl Lösung, einem Arbeitsdruck von 10-100 bar und einem Düsen-Querschnitt von 0,05-0,2 mm unter Zuschaltung einer Hochfrequenz in einem Frequenzbereich von 100-1000 kHz und einer Spannung $U_{eff}$ oberhalb von 1200 Volt , bevorzugt zwischen 1200 und 3000 Volt, entwickelt sich ein Schneidestrahl mit folgenden Eigenschaften: Im Auflösungsbereich des Jet-Strahls ca 5 mm nach Düsenaustritt zündet ein gelblicher Funke zum zu schneidenden Gewebe. Es entsteht ein Schnitt mit scharfen Schnitträndern, koagulierten kleinen Kapilaren und erhaltenen größeren Gefäßen. Die Schnittfläche ist bluttrocken. Dieses Verfahren bedeutet eine Verbesserung der Parenchymchirurgie und eröffnet Möglichkeiten erweiterter chirurgischer Eingriffe an Leber, Pankreas, Milz, Nieren etc.

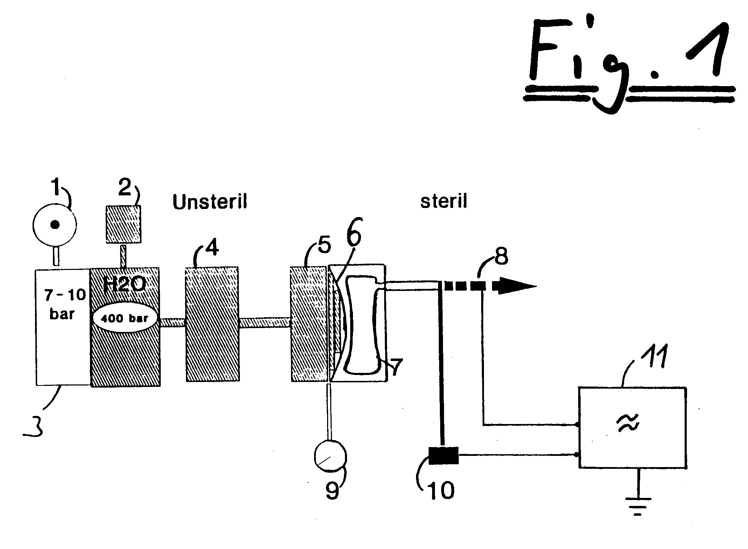

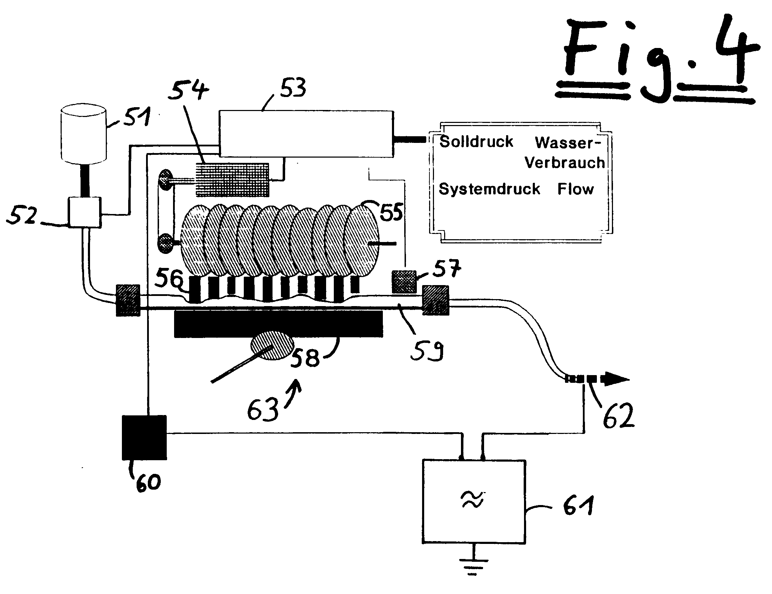

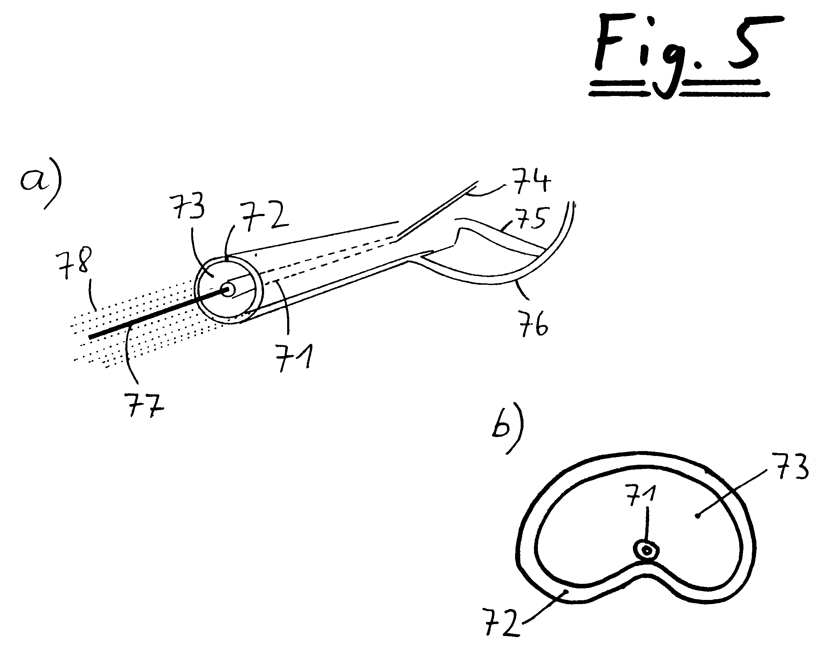

Die Erfindung wird im folgenden unter Bezugnahme auf die Fig.1 bis 5, näher erläutert. Die Fig. 1 bis 4 zeigen verschiedene vorteilhafte Ausbildungen der erfindungsgemäßen Vorrichtung. Fig. 5 zeigt zwei vorteilhafte Ausbildungen der Schneidedüse mit zuätzlicher Absaugung und Spülung.

Alle im Folgenden beschriebenen druckerzeugenden Systeme sind vollständig elektrisch und hygienisch von der Schneidelösung getrennt. Nur so ist eine Zuschaltung eines Hochfrequenzkoagulators möglich, dessen Energie über den feinen Flüssigkeitsstrahl (bevorzugte Parameter: Düsenstärke 0,05-0,2 mm, Druck 10-100 bar) an das zu schneidende Gewebe übertragen wird. Hierzu ist eine elektrisch leitende Flüssigkeit zu verwenden (z.B. NaCl 5-20%). Bei einer Hochfrequenz von 300 kHz und einer Spannung von 2000 Volt konnte experimentell die Übertragung einer Leistung von bis zu 120 Watt nachgewiesen werden.

**Fig. 1**

Die Druckerzeugung erfolgt über eine druckluftgetriebene Hochdruckpumpe 3. Die Pumpe 3 wird über den Luftdruckanschluß 1 mit einem Luftdruck von 7 bis 10 bar angetrieben und erzeugt bis zu 500 bar Druck auf eine unsterile Flüssigkeit z.B. $H_2O$. Der Druck wird über ein Druckausgleichsbehälter 4 in eine Druckkammer 5 geleitet. Die Druckkammer 5 besitzt eine Trennmembran 6, die den Druck auf eine Flüssigkeit überträgt, welche dann als Hochruckflüssigkeitsstrahl über eine Düse 8, die an einem Handstück angebracht ist, zum Schneiden benutzt wird. Die Schneidelösung befindet sich in handelsüblichen Infusionsbeuteln 7, die elektrisch isoliert in der Druckkammer 5 eingebettet sind. Es liegen dadurch zwei getrennte Arbeitskreise vor, von denen jener für die unter Druck stehende Schneideflüssigkeit geschlossen und in sich steril gehalten ist. Auf diese Weise ist ein steriles Arbeiten während einer Operation und zusätzliche Applikation einer elektrischen Hochfrequenz über den Jetstrahl möglich. Durch einen Hochfrequenzgenerator 11, der vorteilhaft eine Strombegrenzung aufweist, wird das elektrisches Hochfrequenzsignal erzeugt, und an das Handstück mit Düse 8 geführt. Das Hochfrequenzsignal wird durch den Fußschalter 10 zugeschaltet. Möglich ist aber auch, den Fußschalter durch einen Ein/Ausschalter, der direkt am Handstück angebracht ist, zu ersetzen.

Mit dem beschriebenen Vorrichtung ist eine Druckerzeugung bis 500 bar erreichbar.

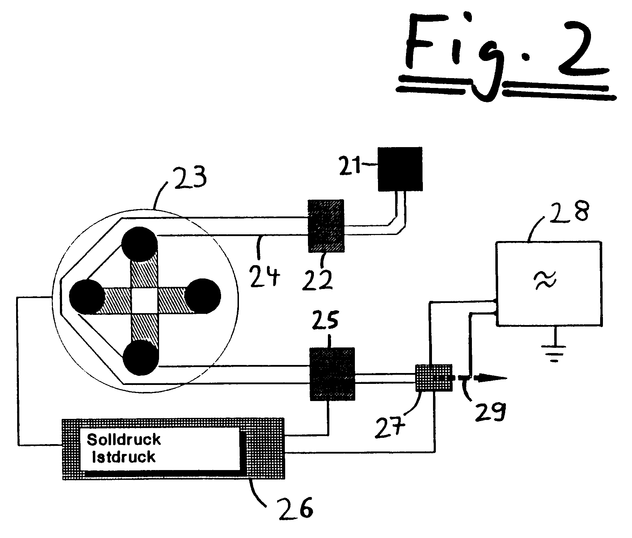

Weitere vorteilhafte Ausbildungen der Erfindung, welche in den Fig. 2 bis 4 dargestellt sind, ergeben sich durch Anwendung einer Peristaltikpumpe zur Druckerzeugung innerhalb der Schneideflüssigkeit. Eine Peristaltikpumpe umfasst allgemein einen flexiblen Druckschlauch, in dem sich das zu beaufschlagende Medium befindet, sowie Mittel (z.B. Pumpenfinger), die zur Druckerzeugung auf diesen Druckschlauch gepreßt werden. Die Schneideflüssigkeit wird in den Vorrichtungen nach Fig. 2 bis Fig. 4 jeweils aus einem Vorratsbehälter über den Druckschlauch der Peristaltikpumpe an die Düse geführt.

**Fig. 2**

Für geringere Drucke bis maximal 100 bar genügt eine vereinfachte Druckerzeugung mit einer Peristaltikpumpe, die in dieser Ausführung als Rollerpumpe 23 ausgebildet ist. Aus einem Vorratsbehälter 21 wird die Jetlösung über den Hochdruckschlauch 24 in die Rollerpumpe 23 und an die Düse am Handstück 29 geführt. Der Arbeitsdruck innerhalb der Jetlösung wird von einem Druckaufnehmer 25, z.B. einem Dehnungsmesser oder Manometer, an einen Rechner 26 gegeben und von dort aus auf einen vorgegebenen Solldruck geregelt. Hier wird der Hochfrequenzstrom des Hochfrequenzgenerators 28 an einem Schalter 27, der unmittelbar am Handstück 29 angeordnet ist, zugeschaltet.

Auch hier ist die Hochdrucktechnik vollständig von der sterilen Jet-Lösung getrennt. Der Austausch der verbrauchten Jet-Lösung ist bei diesem Verfahren einfacher, da hier keine Druckkammer geöffnet werden muss, und somit der Schnitt nicht unterbrochen wird.

**Fig. 3**

Um dem Prinzip der Abtrennung von steriler Jetlösung von der Druckerzeugung gerecht zu werden und gleichzeitig den im Druckschlauch 38 entstehenden Windkesseleffekt steuern zu können, wird eine lineare Peristaltikpumpe 32 eingesetzt, bei der einzeln steuerbare, von Schrittmotoren 39 angetriebene Pumpenfinger 40 zur Druckerzeugung an den Hochdruckschlauch 38 gepreßt werden. Die Schrittmotoren 39 können mit einer elektronischen Steuerungseinrichtung 35 aktiviert werden. Durch eine so gesteuerte Druckerzeugung ist auch ein Pulsbetrieb möglich.

Der Windkesseleffekt führt dazu, daß nach Abschalten der Pumpe 32 der Wasserstrahl erst mit einer Verzögerung abbricht. Mit dieser linearen Peristaltikpumpe können mehrere Pumpenfinger 40 zugleich zurückgesetzt werden, wodurch ein sofortiger Abbruch des Strahls zu erreichen ist. Die Steuerung erfolgt wie in Fig. 2 beschrieben. Der Hochfrequenzstrom des Generators 37 wird an einem Schalter am Handstück 34 zugeschaltet. Alternativ kann auch ein Fußschalter verwendet werden. Mit der Heizvorrichtung 42 kann die Temperatur der Schneideflüssigkeit erhöht werden. Dazu ist sie mit einer Heizspirale und zugehörigem Wärmetauscher verbunden, die am Hochdruckschlauch 38 im Bereich zwischen Pumpe 32 und Düse 34 angebracht sind.

**Fig. 4**

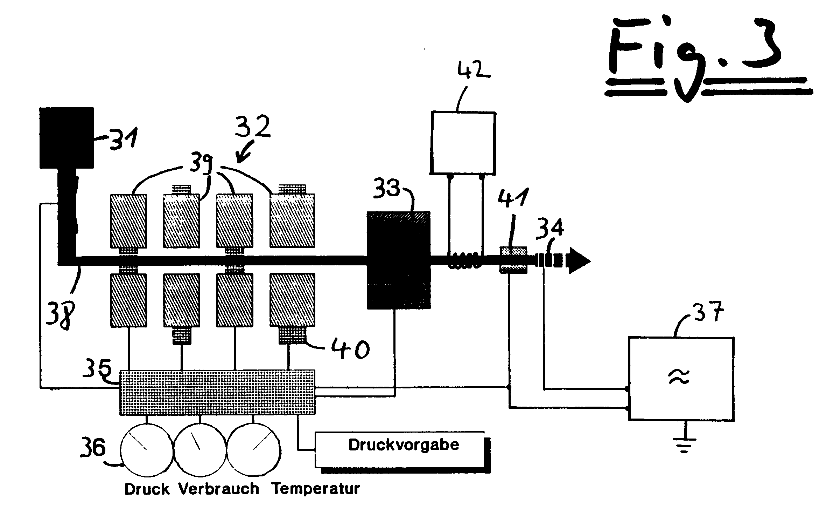

Wie in der Vorrichtung nach Fig. 3 drei, besteht die Druckerzeugungsvorrichtung aus einer Linearpumpe 63, wobei die Jetlösung über einen Hochdruckschlauch 59 zur Düse 62 geführt wird. Hierbei wird eine Schneckenwelle 55 mit einem Motor 54 angetrieben, die ihrerseits Pumpenfinger 56 gegen den Druckschlauch 59 presst. Diese Pumpe wird elektronisch nach vorwählbarem Arbeitsdruck durch einen Computer 53 gesteuert. Ein Pumpenrücklauf sorgt dafür, daß nach Abschaltung der Restdruck, der im Schlauchsystem nach dem Windkesselprinzip noch besteht, abgezogen wird. Die Zuschaltung des Hochfrequenzsignals des Generators 61 sowie der Arbeitsdruck der Jetlösung wird über einen Fußschalter 60 gesteuert. Alternativ kann auch ein Handschalter am Handstück verwendet werden.

Bei allen geschilderten Pumpsystemen ist die elektrische Abkopplung der Jet-Lösung von dem druckerzeugenden Medium verwirklicht. Nur unter dieser Voraussetzung ist eine Zuschaltung von Hochfrequenzenergie von einem Hochfrequenzkoagulator möglich. Um darüberhinaus den Hochfrequenzleckstrom durch den Druckschlauch zur Druckerzeugungsvorrichtung möglichst niedrig zu halten, ist es vorteilhaft, eine Drossel zu verwenden, die mit dem Druckschlauch gewickelt wird oder sonstige HF-Filter zu verwenden. Eine weitere Lösung ist, das Lumen des Druckschlauchs möglichst gering zu halten.

**Fig. 5.**

Die Fig. 5a zeigt eine vorteilhafte Ausführung des Handstücks mit Düse und zusätzlicher Absaugung und Spülung. Die Düse 71, aus der der Schneidestrahl 77 austritt, ist im Zentrum eines Absaugkanals 73 mit kreisförmigen Querschnitt angeordnet. Die Austrittsrichtung der Düse verläuft parallel zur Hauptachse des Absaugkanals. Der Sog im Absaugkanal 73 entsteht durch Unterdruck mit Hilfe einer externen Pumpe (nicht dargestellt). Die Düse 71 ist von der Absaugung elektrisch isoliert. Die feinen, vom Jet-Strahl aufgewirbelten Tröpfchen, werden durch den Sog im Absaugkanal 73 abgesogen.

Die Umfassung des Absaugkanals 73 ist doppelwandig ausgeführt, so daß eine Spülung durch den Innenraum 72 der Umfassung geführt werden kann. Der Innenraum 72, im folgenden als Spülkanal bezeichnet, weist einen ringförmigen Querschnitt auf. Die aus dem Spülkanal 72 austretende Spülflüssigkeit 78 wirkt wie ein Wasservorhang, so daß die feinen vom Jet-Strahl 77 aufgewirbelten Tröpfchen niedergehalten werden. Die Spülflüssigkeit erlaubt außerdem eine Verdünnung der Jet-Lösung. Über den Absaugkanal 73 kann die Spül- und Jet-Flüssigkeit abgesaugt werden.

Neben dem hier gezeigten Handstück sind für den Querschnitt des Absaugkanals sowie für die Anordnung der Düse innerhalb des Absaugkanals auch andere zweckmäßige Ausführungen möglich. Ein Beispiel hierfür zeigt Fig. 5b. Der Absaugkanal 73 ist hier von etwa nierenförmigen Querschnitt, wobei die Düse 71 an der Wandung des Absaugkanals 73 anliegt. Wie in Fig. 5a ist die Umfassung des Absaugkanals 73 doppelwandig ausgeführt, so daß der Innenraum 72 einen Spülkanal bildet.

In einer weiteren vorteilhaften Ausführung (in der Fig. nicht gezeigt) umfaßt das Handstück einen Laser sowie ein Gebläse oder ein Heißluftgebläse.

Für den Einsatz in der minimal invasiven Chirurgie kann das Handstück miniatisiert werden, so daß es auch über endoskopische Arbeitskanäle in den Körper eingeführt werden kann.

**Bezugsziffern zu den Fig. 1 bis 5**

**Fig. 1**

1. Luftdruckanschluss
2. Vorratbehälter
3. Hochdruckpumpe
4. Druckausgleichsbehälter
5. Druckkammer
6. Trennmembran
7. Schneidelösungsbehälter mit Lösung
8. Handstück mit Düse
9. Manometer zur Druckregelung
10. Fußschalter
11. Hochfrequenzgenerator

**Fig. 2**

21. Vorratsbehälter für die Schneidelösung
22. Halterung des Peristaltikschlauches
23. Rollerpumpe
24. Hochdruckschlauch
25. Druckaufnehmer
26. Rechner mit Monitor
27. Schalter
28. Hochfrequenzgenerator
29. Handstück mit Düse

**Fig. 3**

31. Vorratsbehälter für die Schneidelösung
32. Schrittmotorpumpe
33. Druckaufnehmer
34. Handstück mit Düse
35. Rechner mit Druck und Flussvorgabe
36. Monitor
37. Hochfrequenzgenerator
38. Hochdruckschlauch
39. Schrittmotor
40. Pumpenfinger
41. Schalter

42. Heizvorrichtung

**Fig. 4**

51. Vorratsbehälter für die Schneideflüssigkeit
52. Flussmesser
53. Rechner
54. Motor
55. Schneckenwelle
56. Pumpenfinger
57. Druckaufnehmer
58. Andruckschiene
59. Hochdruckschlauch
60. Fußschalter
61. Hochfrequenzgenerator
62. Handstück mit Düse
63. Fingerpumpe

**Fig. 5**

71. Abgeschirmte Düse
72. Ringförmige Spülung
73. Nach zentral gerichtete Absaugung
74. Zuführungsschlauch der Schneidelösung
75. Absaugungsschlauch
76. Zuführungsschlauch der Spüllösung
77. Schneidestrahl
78. Spülungsring

**Patentansprüche**

1. Verfahren zum Schneiden organischer Gewebe wobei eine elektrisch leitfähige Schneideflüssigkeit durch eine Druckerzeugungsvorrichtung (3) auf einen bestimmten Arbeitsdruck gebracht wird und die druckbeaufschlagte Schneideflüssigkeit als Schneidestrahl aus einer Düse austritt, **dadurch gekennzeichnet**, daß

   - die Schneideflüssigkeit elektrisch und hygienisch getrennt von der Druckerzeugungsvorrichtung geführt wird und
   - dem Schneidestrahl eine elektrische Hochfrequenz aufgeprägt wird.

2. Verfahren nach Anspruch 1, **dadurch gekennzeichnet**, daß dem Schneidestrahl eine elektrische Frequenz zwischen 100 bis 1000 kHz bei einer Spannung oberhalb von 1200 Volt aufgeprägt wird.

3. Verfahren nach einem der vorangehenden Ansprüche, **dadurch gekennzeichnet**, daß die Düsenöffnung einen Durchmesser von 0,05 bis 0,2 mm aufweist.

4. Verfahren nach einem der vorangehenden Ansprüche, **dadurch gekennzeichnet**, daß mit Hilfe eines Regelkreises (9) der Druck der Schneideflüssigkeit auf einen vorbestimmten Arbeitsdruck eingestellt wird.

5. Verfahren nach einem der vorangehenden Ansprüche, **dadurch gekennzeichnet**, daß über eine Durchfluß- und Druckmessung ein Sicherheitsystem gesteuert wird, das Düsenverschüsse und Leckagen meldet und gegebenenfalls die Druckerzeugung auf Null setzt.

6. Verfahren nach einem der vorangehenden Ansprüche, **dadurch gekennzeichnet**, daß die Schneideflüssigkeit erwärmt wird.

7. Flüssigkeitsstrahlschneideinstrument zum Schneiden organischer Gewebe mit

   - einer Druckerzeugungsvorrichtung (3) zur Druckbeaufschlagung einer elektrisch leitfähigen Schneideflüssigkeit
   - einem Handstück (8) mit einer Düse, aus der die druckbeaufschlagte Schneideflüssigkeit austritt
   - einem elektrischen Hochfrequenzgenerator (11) zur Koagulation,
     **dadurch gekennzeichnet**, daß
   - die Druckerzeugunsvorrichtung (3) elektrisch und hygienisch von der Schneideflüssigkeit getrennt ist
     und
   - dem Schneidestrahl das Signal des Hochfrequenzgenerators (11) aufgeprägt wird.

8. Vorrichtung nach Anspruch 7, **dadurch gekennzeichnet**, daß die Druckerzeugungsvorrichtung eine mit Luftdruck beaufschlagte Hochdruckpumpe (3) umfaßt, die in einem Druckmedium den notwendigen Arbeitsdruck erzeugt, und der Druck in dem Druckmedium über eine in einer Druckkammer (5) befindlichen Membran (6) an die Schneideflüssigkeit übertragen wird.

9. Vorrichtung nach einem der vorangehenden Ansprüche 7 oder 8, **dadurch gegennzeichnet**, daß die Druckerzeugungsvorrichtung eine Peristaltikpumpe (23) ist, enthaltend einen flexiblem Druckschlauch (24), sowie Mittel (40), die zur Druckerzeugung gegen diesen Druckschlauch gepreßt werden, wobei die Schneideflüssigkeit aus einem Vorratsbehälter (21) über den flexiblen Druckschlauch der Peristatitkpumpe an die Düse geführt wird.

10. Vorrichtung nach Anspruch 9, **dadurch gekennzeichnet**, daß die Peristaltikpumpe eine Rollerpumpe (23) ist.

11. Vorrichtung nach Anspruch 9, **dadurch gekennzeichnet**, daß die Druckerzeugungsvorrichtung eine lineare Peristaltikpumpe (32) ist, bei der mehrere, mit Schrittmotoren (39) einzeln steuerbare Pumpenfinger (40) auf den Druckschlauch (38) gepreßt werden.

12. Vorrichtung nach Anspruch 9, **dadurch gekennzeichnet**, daß die Druckerzeugungsvorrichtung eine lineare Peristaltikpumpe (63) ist, die eine motorgetriebene Schneckenwelle (55) umfaßt, wobei die Schneckenwelle (55) Pumpenfinger (56) gegen den Druckschlauch (59) preßt.

13. Vorrichtung nach einem der vorangehenden Ansprüche 7 bis 12 **dadurch gekennzeichnet**, daß die Druckerzeugungsvorrichtung (63) am Handstück oder mit einem Fußschalter (60) geschaltet werden kann.

14. Vorrichtung nach einem der vorangehenden Ansprüche 7 bis 13, **dadurch gekennzeichnet**, daß der Hochfrequenzgenerator (61) am Handstück oder mit einem Fußschalter (60) geschaltet werden kann.

15. Vorrichung nach einem der vorangehenden Ansprüche 7 bis 14, **dadurch gekennzeichnet**, daß das Handstück eine Absaugung umfaßt.

16. Vorrichtung nach einem der vorangehenden Ansprüche 7 bis 15, **dadurch gekennzeichnet**, daß das Handstück eine Spülung umfaßt.

17. Vorrichtung nach einem der vorangehenden Ansprüche 15 oder 16, **dadurch gekennzeichnet**, daß die Düse (71) innerhalb eines Absaugkanals (73) angeordnet ist.

18. Vorrichtung nach einem der vorangehenden Ansprüche 15 bis 17, **dadurch gekennzeichnet**, daß die Umfassung des Absaugkanals (73) doppelwandig ausgebildet ist, wobei das Innere (72) der Umfassung als Spülkanal genutzt wird.

**Claims**

1. Method for cutting organic tissues, in which an electrically conductive cutting liquid is brought to a specific working pressure by a pressure-generating device (3), and the pressurized cutting liquid emerges as a cutting jet from a nozzle, characterized in that

   - the cutting liquid conveyed is electrically and hygienically isolated from the pressure-generating device, and
   - an electrical high frequency is imposed on the cutting jet.

2. Method according to Claim 1, characterized in that an electrical frequency of between 100 and 1000 kHz is imposed on the cutting jet at a voltage of over 1200 volts.

3. Method according to one of the preceding claims, characterized in that the nozzle opening has a diameter of 0.05 to 0.2 mm.

4. Method according to one of the preceding claims, characterized in that the pressure of the cutting liquid is set to a predetermined working pressure with the aid of a control loop (9).

5. Method according to one of the preceding claims, characterized in that a safety system, which is controlled via flow measurement and pressure measurement, indicates nozzle blockages and leakages and optionally sets the pressure generation to zero.

6. Method according to one of the preceding claims, characterized in that the cutting liquid is heated.

7. Liquid jet cutting instrument for cutting organic tissues, with

    - a pressure-generating device (3) for pressurizing an electrically conductive cutting liquid,
    - a handpiece (8) with a nozzle from which the pressurized cutting liquid emerges,
    - an electrical high-frequency generator (11) for coagulation,

    characterized in that

    - the pressure-generating device (3) is electrically and hygienically isolated from the cutting liquid, and
    - the signal of the high-frequency generator (11) is imposed on the cutting jet.

8. Device according to Claim 7, characterized in that the pressure-generating device comprises an air-pressurized high-pressure pump (3) which generates the necessary working pressure in a pressure medium, and the pressure in the pressure medium is transmitted to the cutting liquid via a membrane (6) located in a pressure chamber (5).

9. Device according to one of the preceding Claims 7 and 8, characterized in that the pressure-generating device is a peristaltic pump (23) containing a flexible pressure hose (24), and means (40) which are pressed against this pressure hose in order to generate pressure, the cutting liquid being conveyed to the nozzle from a storage container (21) via the flexible pressure hose of the peristaltic pump.

10. Device according to Claim 9, characterized in that the peristaltic pump is a roller pump (23).

11. Device according to Claim 9, characterized in that the pressure-generating device is a linear peristaltic pump (32) in which a plurality of pump fingers (40), which can be controlled individually with stepping motors (39), are pressed onto the pressure hose (38).

12. Device according to Claim 9, characterized in that the pressure-generating device is a linear peristaltic pump (63) which comprises a motor-driven worm shaft (55), said worm shaft (55) pressing pump fingers (56) against the pressure hose (59).

13. Device according to one of the preceding Claims 7 to 12, characterized in that the pressure-generating device (63) can be switched at the handpiece or with a foot switch (60).

14. Device according to one of the preceding Claims 7 to 13, characterized in that the high-frequency generator (61) can be switched at the handpiece or with a foot switch (60).

15. Device according to one of the preceding Claims 7 to 14, characterized in that the handpiece comprises a suction system.

16. Device according to one of the preceding Claims 7 to 15, characterized in that the handpiece comprises a flushing system.

17. Device according to one of the preceding Claims 15 and 16, characterized in that the nozzle (71) is arranged inside a suction channel (73).

18. Device according to one of the preceding Claims 15 to 17, characterized in that the enclosure of the suction channel (73) is designed with a double wall, with the interspace (72) of the enclosure being used as a flushing channel.

**Revendications**

1. Procédé de coupe de tissus organiques, un liquide de coupe conducteur de l'électricité étant amené par un dispositif générateur de pression (3) à une pression de travail déterminée et le liquide de coupe sous pression sortant d'une buse sous la forme d'un jet de coupe, caractérisé par le fait
    que le liquide de coupe est conduit de façon électriquement et hygiéniquement séparée du dispositif générateur de pression, et
    qu'une haute fréquence électrique est superposée au jet de coupe.

2. Procédé suivant la revendication 1, caractérisé par le fait qu'une fréquence électrique comprise entre 100 et 1000 kHz sous une tension supérieure à 1200 volts est superposée au jet de coupe.

3. Procédé suivant l'une des revendications précédentes, caractérisé par le fait que l'orifice de la buse présente un diamètre de 0,05 à 0,2 mm.

4. Procédé suivant l'une des revendications précédentes, caractérisé par le fait qu'à l'aide d'un circuit de réglage (9), la pression du liquide de coupe est établie à une pression de travail prédéterminée.

5. Procédé suivant l'une des revendications précédentes, caractérisé par le fait qu'un système de sécurité commandé par une mesure de débit et de pression signale les obstructions de la buse et les fuites et, le cas échéant, met à zéro la génération de pression.

6. Procédé suivant l'une des revendications précédentes, caractérisé par le fait que le liquide de coupe est chauffé.

7. Instrument de coupe à jet de liquide pour la coupe de tissus organiques, comprenant
   un dispositif générateur de pression (3) pour mettre sous pression un liquide de coupe conducteur de l'électricité,
   une pièce à main (8) avec une buse de sortie du liquide de coupe sous pression, et
   un générateur électrique haute fréquence (11) pour la coagulation,
   caractérisé par le fait
   que le dispositif générateur de pression (3) est séparé électriquement et hygiéniquement du liquide de coupe, et
   que le signal du générateur haute fréquence (11) est superposé au jet de coupe.

8. Dispositif suivant la revendication 7, caractérisé par le fait que le dispositif générateur de pression comprend une pompe haute pression (3) alimentée en air comprimé qui produit la pression de travail nécessaire dans un fluide de pression, et que la pression dans le fluide de pression est transmise au liquide de coupe en passant par une membrane (6) se trouvant dans une chambre de pression (5).

9. Dispositif suivant l'une des revendications précédentes 7 ou 8, caractérisé par le fait que le dispositif générateur de pression est une pompe péristaltique (23) comprenant un tuyau de pression (24) flexible, ainsi que des moyens (40) qui sont pressés contre ce tuyau en vue de la génération de pression, le liquide de coupe étant amené d'un réservoir (21) à la buse en passant par le tuyau flexible de la pompe péristaltique.

10. Dispositif suivant la revendication 9, caractérisé par le fait que la pompe péristaltique est une pompe rotative à rouleaux.

11. Dispositif suivant la revendication 9, caractérisé par le fait que le dispositif générateur de pression est une pompe péristaltique (32) linéaire dans laquelle plusieurs doigts de pompe (40) susceptibles d'être commandés individuellement à l'aide de moteurs pas à pas (39) sont pressés contre le tuyau (38).

12. Dispositif suivant la revendication 9, caractérisé par le fait que le dispositif générateur de pression est une pompe péristaltique (63) linéaire qui comprend un arbre à vis (55) entraîné par moteur, l'arbre à vis (55) pressant des doigts de pompe (56) contre le tuyau de pression (59).

13. Dispositif suivant l'une des revendications précédentes 7 à 12, caractérisé par le fait que le dispositif générateur de pression (63) peut être commandé sur la pièce à main ou sur une pédale (60).

14. Dispositif suivant l'une des revendications précédentes 7 à 13, caractérisé par le fait que le générateur haute fréquence (61) peut être commandé sur la pièce à main ou à l'aide d'une pédale (60).

15. Dispositif suivant l'une des revendications précédentes 7 à 14, caractérisé par le fait que la pièce à main comprend une aspiration.

16. Dispositif suivant l'une des revendications précédentes 7 à 15, caractérisé par le fait que la pièce à main comprend un rinçage.

17. Dispositif suivant l'une des revendications précédentes 15 ou 16, caractérisé par le fait que la buse (71) est disposée à l'intérieur d'un canal d'aspiration (73).

18. Dispositif suivant l'une des revendications précédentes 15 à 17, caractérisé par le fait que le canal d'aspiration (73) est réalisé à double paroi, l'intervalle (72) entre la double paroi étant utilisé comme canal de rinçage.

$\underline{\underline{Fig. 1}}$

Fig. 2

Solldruck
Istdruck

Fig. 3

Fig.4

| Solldruck | Wasser-Verbrauch |
| Systemdruck | Flow |

EP 0 555 549 B1

Fig. 5

a)

74
75
73  72
78
76
71
77

b)

71  73
72

EP 0 555 549 B1